Europäisches Patentamt

European Patent Office

Office européen des brevets

(11) Publication number: **0 166 359**
**B1**

(12) **EUROPEAN PATENT SPECIFICATION**

(45) Date of publication of patent specification: **01.08.90**

(21) Application number: **85107509.3**

(22) Date of filing: **18.06.85**

(51) Int. Cl.⁵: **C 07 C 11/08,** C 07 C 11/09,
C 07 C 5/333

(54) **Recycle of butadiene product in catalytic dehydrogenation process.**

(30) Priority: **19.06.84 US 622311**

(43) Date of publication of application:
**02.01.86 Bulletin 86/01**

(45) Publication of the grant of the patent:
**01.08.90 Bulletin 90/31**

(84) Designated Contracting States:
**AT BE CH DE FR GB IT LI LU NL SE**

(56) References cited:
US-A-3 692 701
US-A-3 726 942
US-A-3 737 473

(73) Proprietor: **PHILLIPS PETROLEUM COMPANY
5th and Keeler
Bartlesville Oklahoma 74004 (US)**

(72) Inventor: **Olbrich, Michael Eugene
421 N.E. Spruce
Bartlesville, OK 74006 (US)**
Inventor: **Brinkmeyer, Francis Maurice
2124 S.E. Skyline Dr.
Bartlesville, OK 74006 (US)**

(74) Representative: **Dost, Wolfgang, Dr.rer.nat.,
Dipl.-Chem. et al
Patent- und Rechtsanwälte Bardehle-
Pagenberg-Dost-Altenburg & Partner Postfach
86 06 20
D-8000 München 86 (DE)**

Courier Press, Leamington Spa, England.

**Description**

Background of the invention

In a process for dehydrogenation of butane feedstock in which both isobutane and n-butane are present there are produced the desirable butenes and isobutene and there is also produced a by-product of butadiene. The real problem with some of the processes currently used is that there is also a large amount of unconverted butane feedstock that passes through into the product. For a process to be most efficient there needs to be a separation of the product butenes and isobutene from the feedstock butane so that the butane can be recycled to the reaction in consecutive passes over the catalyst. Unfortunately, however, butadiene produced as a by-product is not easily separated from the butane in the product mixture so that any feedstock for recycle is contaminated with the butadiene which is a well known coke producer in dehydrogenation processes.

A logical way to overcome the presence of this contaminant by-product in the recycle feedstock separated from the dehydrogenation product is to provide a hydrotreating system which rehydrogenates the butadiene before it is passed into the dehydrogenation reaction. It has now been discovered, however, that using the preferred catalyst as set out below the dehydrogenation reaction itself acts to maintain only a low level of butadiene in the product stream even though recycle containing butadiene is returned directly to the dehydrogenation reaction. It has been proposed as explanation of these results that the butadiene is rehydrogenated probably with some structural isomerization during the dehydrogation reaction.

It is therefore an object of this invention to provide a method for dehydrogenation using a catalyst that permits direct recycle of butadiene contaminant through the reaction system.

Other aspects, objects and the various advantages of this invention will become apparent upon study of this disclosure and the appended claims.

Statement of the invention

In accordance with this invention, a method is provided for dehydrogenation of a feedstock of isobutane and n-butane to produce butenes, isobutene and butadiene in which the feedstock is contacted in a reaction zone with a steam active dehydrogenation catalyst which is (a) a calcined support of at least one aluminate spinel composition, (b) at least one Group VIII metal, (c) with or without at least one Group IA metal and (d) at least one tin group metal selected from among tin, germanium, and lead under dehydrogenation conditions thereby producing a product stream containing isobutane, n-butane, butenes, isobutene, and butadiene; separating the product stream to provide a separated product containing butenes and isobutene and a recycle feedstock composition containing isobutane, n-butane, butadiene and residual butenes; and recycling the recycled feedstock along with fresh feedstock to the reaction zone in a combined feedstock that has up to 20 mol percent n-butane and up to about 5 mol percent butadiene thereby producing a combined product stream having an amount of butadiene substantially equivalent to the amount of butadiene produced without the presence of butadiene in the feedstock.

In another embodiment of the invention, the combined product stream produced by recycling the recycled feedstock along with fresh feedstock through the reaction zone is separated to produce a recycle feedstock composition containing isobutane, n-butane and butadiene which is then recycled along with fresh feedstock to the reaction zone to produce a stream containing isobutane, n-butane, butenes, isobutene and butadiene with the repeating of these steps to obtain the desired amount of product.

In another embodiment of the invention a process is provided for producing butenes and isobutene from a feedstock that contains isobutane, n-butane and butadiene. In the process the feedstock is contacted with a steam active dehydrogenation catalyst under dehydrogenation conditions to produce a product containing butenes, isobutene, isobutane, n-butane and an amount of butadiene substantially equivalent to that in the feedstock.

It has been found that highly active and exceptionally selective catalysts for the dehydrogenation of steam-diluted alkanes, cycloalkanes, and arylalkanes can be prepared by highly calcining a Group II aluminate spinel support. These Group II aluminate spinels are particularly effective and excellent results are obtained with zinc aluminate spinel. A catalyst composition is then formed by combining the highly calcined support with a metal selected from the group consisting of nickel, platinum, palladium, ruthenium, irridium, rhodium, osmium, and mixtures thereof. Other catalyst compositions are then formed by combining with this composition a material selected from the group consisting of a Group IA metal and a tin group metal and mixtures thereof.

The groups of metals referred to herein are as classified in the Periodic Table published in the Chemical Rubber Company's "Handbook of Chemistry and Physics", 45th Edition (1964), page B-2.

Aluminate spinels, as referred to herein, are compounds of the formula $M(AlO_2)_2$ or $MO \cdot Al_2O_3$ wherein M is a Group IIA or IIB metal with a valence of 2 such as Zn, Mg, Be and Ca.

The highly calcined supports useful in this invention can be prepared by calcining for a sufficient time and at a sufficient temperature Group II aluminate spinels. The spinels can be prepared by any known method and in a preferred embodiment are prepared by the coprecipitation method wherein appropriate amounts of suitable aluminum compounds and suitable Group II metal compounds are combined under spinel-forming conditions. It is preferred that the proportions of these ingredients be approximately stoichiometric or, alternatively, that the Group II metal compound be up to 10 percent or more in excess of stoichiometric.

The highly calcined support is prepared, preferably, by calcination for 1—100 hours at 816—1371°C (1500—2500°F). In another embodiment, a highly calcined support is produced by calcining the support, preferably, from 2—50 hours at 1093—1371°C (2000—2500°F). In a more preferred embodiment, the support is calcined by heating for about 6 hours at about 871°C (1600°F).

The first catalyst composition of this invention can be prepared by combining, in any manner known to the art, certain Group VIII metals, or metal compounds capable of reduction to the metal, including nickel, platinum, ruthenium, palladium, iridium, rhodium, osmium, and mixtures thereof, with the highly calcined support of this invention. Platinum, which is very effective, is preferred. The Group VIII metal content of the catalyst can be in the range of 0.01—5 weight percent of the support and, in one embodiment, is in the range of 0.1—1 weight percent of the support. Throughout this application the term "weight percent of the support" means parts by weight per 100 parts by weight of support.

Any platinum group metal compound that produces the desired results can be used. In the discussion of the compounds that can be used the platinum compounds will be used as nonlimiting examples. It is to be understood that similar compounds of the other platinum group metals can be used. Examples of simple or non-coordination compounds that can be used are platinic chloride, chloroplatinic acid and ammonium chloroplatinate. Examples of coordination platinum compounds that can be used are: platinum aminoacetate, platinum dimethyl dioxime, tetraamineplatinum hydroxide, platinum diamine dinitrate, platinum tetraamine dihydroxide, platinum diamine dihydroxide, platinum hexamine dihydroxide, platinum hexamine tetrahydroxide, platinum diamine tetrahydroxide, platinum diamine dihydroxide dinitrate, platinum diamine tetranitrate, platinum diamine dinitrite, platinum tetraamine dicarbonate and platinum diamine oxalate. Additionally many complex or coordination divalent and tetravalent platinum compounds are known and can be used.

When added to the support by impregnation from solution, some of the compounds can be added from aqueous solution, but others will require non-aqueous solvents such as alcohols, hydrocarbons, ethers and ketones.

The second catalyst composition of this invention is prepared by combining a Group IA metal or metal compound with the components of the first composition with or without a tin group metal. This can be conveniently done by conventional impregnation. The amount of each alkali metal compound or combination of compounds can exist in the range of 0.01—5 weight percent of the total catalyst; however, in one embodiment, a range of 0.1—1 weight percent of said support is used. Any Group IA metal in compound form is suitable for use in this invention. Other convenient compounds are, e.g. the carbonates, acetates and hydroxides of, e.g. sodium, barium, potassium and calcium.

The third catalyst composition of this invention is prepared by combining a tin group metal including lead, tin and germanium with the components of the first catalyst composition with or without a Group IA metal. The tin group metal can be incorporated in the composition in the range of 0.01—5 weight percent of said support and, in one embodiment, can be incorporated in the composition in the range of 0.1—1 weight percent of said support. Although any tin group metal in compound form is fully within the scope of this invention, some convenient tin group compounds are the halides, nitrates, oxalates, acetates, carbonates, propionates, tartrates, bromates, chlorates, oxides and hydroxides of tin, germanium and lead. Tin, itself, is the preferred tin group metal and impregnation of the supports with tin compounds such as the stannous halides is particularly effective and convenient.

Generally speaking, the Group IA, VIII and tin group compounds, which are combined with the calcined supports to form the improved catalysts of the present invention, can be sequentially combined with the support, in any order, or for convenience, can be applied simultaneously in a single impregnation operation. After impregnation, the composite solids are dried and calcined.

The catalyst systems of this invention are conveniently employed at temperatures from 399 to 677°C (750 to 1250°F)., preferably from 538—593°C (1000 to 1100°F), and at total pressures in the range of 0 to 3.45 MPa gauge (0 to 500 psig), preferably 0 to 1.73 MPa gauge (0 to 250 psig). Steam to hydrocarbon mol ratios of 0.5:1 to 30:1, preferably 2.5:1 to 15:1, are employed. Liquid hourly space velocity (LHSV) of hydrocarbon feed is in the range of 2.0 to 6.0 volumes HC per hr per volume of catalyst preferably 3.5 to 4.5 $h^{-1}$. The process cycle is 0.5 to 24 h and the regeneration period is 0.5 to 4.0 hours. The catalyst is regenerated with steam diluted air such that the $O_2$ content of the regeneration gas mixture is 0.5 to 4.0 mol percent. The regeneration treatment can be carried out at temperatures and pressures within said dehydrogenation operating ranges.

The separation of the product stream to provide butenes and isobutene as product and a stream containing butanes and butadiene as recycle for the reaction zone is well known in the art. The usual form of separation is fractionation which does not allow the separation of the butadiene contaminant from the isobutane and n-butane. The recycle stream is then combined with an amount of fresh feedstock to provide a composition of feedstock that is equivalent to the original feedstock with the exception of the addition of butadiene contaminant.

The process of reacting the combined feedstock, separating the product stream, and recycling the separated recycle material to the reaction is repeated as desired.

The following example describes the basic process of the invention.

Example

To illustrate that in the process of this invention the butadiene contaminant will have so small effect as to go unnoticed in the reaction of the recycled separated reaction product a series of tests was conducted in which 95 mol percent isobutane feed was spiked or deliberately increased with one percent butadiene.

A slurry consisting of distilled water, finely divided alumina, finely divided reagent grade zinc oxide and finely divided reagent stannic oxide was ball milled for one hour to obtain an intimate mixture. The slurry was dried overnight at 93 to 104°C (200—220°F) in a forced draft oven. The resulting dry cake was crushed, sieved to remove coarse particles and the powder was compounded with 8 weight percent of a polyethylene lubricant. The mixture was formed into 0.32 cm (1/8-inch) pellets and calcined in air in a muffle furnace which was programmed as follows: 2 hours at 427°C (800°F), 2 hours at 593°C (1,100°F) and 3 hours at 1010°C (1,850°F).

Thus prepared tin-containing support had a surface area of 12 square meters per gram, a pore volume of 0.48 ml per gram. The support contained 35 weight percent zinc, 26.1 weight percent aluminum, 1.0 weight percent tin and 57.9 weight percent combined oxygen.

A portion of the tin-containing catalyst support the above calcined slurry was impregnated with platinum from an aqueous solution of chloroplatinic acid to form a catalyst composition containing 0.6 weight percent platinum based on the weight of the final catalyst. The mixture was dried 3 hours at 110°C (230°F) and calcined 3 hours at 593°C (1100°F).

Alternating runs with the spiked and regular isobutane feed were made. The conversion of isobutane and the selectivity to isobutane through the cycle are illustrated in Tables I and II below which sets out typical results with and without the butadiene in the feedstock.

Table I gives test results obtained early in the runs (1.3 and 1.5 hours) while Table II gives test results obtained later in the runs (5.4 and 5.5 hours) for the not spiked and spiked with butadiene in the feed hydrocarbon.

TABLE I
Product Distribution

All concentrations shown are C4 equivalent mole percent.

| Products | Not spiked with butadiene | Spiked with 1 mol% butadiene |
|---|---|---|
| | Run 2201 1.32 hours into cycle | Run 2202 1.50 hours into cycle |
| C5+ | 0.0 | 0.0 |
| C2/C2= | 0.2 | 0.2 |
| CO2 | 1.8 | 1.8 |
| C3 | 1.1 | 1.1 |
| C3= | 0.7 | 0.6 |
| iC4 | 44.4 | 44.2 |
| nC4 | 2.3 | 2.6 |
| iC4= | 46.4 | 46.2 |
| C4=1 | 0.8 | 0.8 |
| tC4=2 | 0.9 | 1.0 |
| cC4=2 | 0.6 | 0.7 |
| 1,3 C4== | 0.0 | 0.2 |
| C1 | 0.6 | 0.6 |
| C0 | 0.0 | 0.0 |
| | | |
| Feed | | |
| iC4 | 95.0 | 94.9 |
| nC4 | 4.8 | 4.0 |
| C3 | 0.2 | 0.2 |
| 1,3 C4== | 0.0 | 0.9 |
| | | |
| Conversion of isobutane | 53.2% | 53.4% |
| Selectivity to isobutene | 92.2% | 91.1% |
| Conversion of butadiene | 0% | 77.8% |

# EP 0 166 359 B1

Conditions of all runs of Table I and II

Liquid hourly space velocity,
vol/hr feed/vol catalyst — 4.0
Mols steam/mol hydrocarbon — 5.0
Average bed temperature °C (°F) — 566 (1050)
Average pressure MPa (psig) — 0.35 (50)

## TABLE II
### Product distribution

| Products | Not spiked run 2203 5.4 hours into cycle | Spiked run 2205 5.5 hours into cycle |
|---|---|---|
| C5+ | 0.0 | 0.0 |
| C2/C2= | 0.1 | 0.1 |
| CO2 | 1.1 | 1.1 |
| C3 | 0.7 | 0.7 |
| C3= | 0.5 | 0.5 |
| iC4 | 49.8 | 48.6 |
| nC4 | 2.4 | 2.6 |
| iC4= | 42.8 | 43.4 |
| C4=1 | 0.7 | 0.8 |
| tC4=2 | 0.8 | 0.9 |
| cC4=2 | 0.6 | 0.7 |
| 1,3 C4== | 0.1 | 0.2 |
| C1 | 0.4 | 0.4 |
| C0 | 0.0 | 0.0 |
| Feed | | |
| iC4 | 95.0 | 94.9 |
| nC4 | 4.8 | 4.0 |
| C3 | 0.2 | 0.2 |
| 1,3 C4== | 0.0 | 0.9 |
| Conversion of isobutane | 47.5% | 48.8% |
| Selectivity to isobutene | 94.8 mol% | 93.8 mol% |
| Conversion of butadiene | 0 mol% | 77.8 mol% |

In the run, performance through the cycle was virtually unchanged. Had the butadiene been converted directly or extensively to coke in the reactor a rapid decline in conversion would be expected or a dramatic increase in carbon dioxide in the reactor effluent could be seen. Neither of these results were observed. It can be concluded, therefore, that the butadiene was not being converted extensively to coke in the reactor.

Since 77.8% of the butadiene fed to the reaction zone was converted there are two possibilities as to what happened to it: either the butadiene was being rehydrogenated or the butadiene was coking in the preheaters before the reactor. Table I shows the product distributions for a regular and a spiked feed run. Inspection of these data show that the normal butane, trans-butane-2 and cis-butane-2 concentrations were all higher in the spiked effluent than in the regular effluent even though the spiked feed contained less normal butane. This indicates that some rehydrogenation was occurring. It cannot be directly determined if all of the converted butadiene became normal butane products because some skeletal isomerization could occur, however, it is evident that at least the majority of the butadiene was rehydrogenated. While butadiene is generally considered to be a coke precursor, in the environment of the present reaction near the reactor exit there is a 700:1 hydrogen to butadiene molar ratio which shifts the equilibrium away from coke and towards the formation n-butenes.

Additionally, there was no indication of increased pressure drop across the coils of the preheater which would indicate coking in the preheaters and the evidence showed little or no coke formation on the catalyst itself that could permanently deactivate it.

## Claims

1. A method for dehydrogenation of the feedstock of isobutane and n-butane to produce butenes, isobutene, and butadiene, said method being characterized by
(1) contacting the said feedstock in a reaction zone with steam and a steam active dehydrogenation

5

catalyst comprised of a calcined support of at least one aluminate spinel composition, at least one Group VIII metal, with or without at least one Group IA metal and at least one tin group metal selected from tin, germanium, and lead under dehydrogenation conditions thereby producing a product stream comprising isobutane, n-butane, butenes, isobutene, and butadiene;

(2) separating said product stream to provide a separated product comprising butenes and isobutene and a recycle feedstock composition comprising isobutane, n-butane, and butadiene and residual butenes; and

(3) recycling said recycle feedstock along with fresh feedstock to the reaction zone in a combined feedstock that has up to 20 mol percent n-butane and up to 5 mol percent butadiene thereby producing a combined product stream having an amount of butadiene substantially equivalent to the amount of butadiene produced without the presence of the butadiene of the feedstock.

2. The method of claim 1 characterized by further comprising:

(4) separating said combined product stream to produce a recycle feedstock composition comprising isobutane, n-butane and butadiene and residual butenes and a product containing butenes and isobutane; and

(5) recycling said recycle feedstock composition along with fresh feedstock to the reaction zone to produce a stream comprising isobutane, n-butane, butenes, isobutene and butadiene and residual butenes.

3. The method of claim 2 characterized by repeating seriatim steps (4) and (5) until the desired amount of product is obtained.

4. The method of claim 1 characterized in that said calcined support is zinc aluminate spinel, said Group VIII metal is platinum, and said tin group metal is tin.


**Patentansprüche**

1. Verfahren zur Dehydrierung eines Ausgangsmaterials aus Isobutan und n-Butan einer Bildung von Butenen, Isobuten und Butadien, gekennzeichnet durch

(1) Kontaktieren des Ausgangsmaterials in einer Reaktionszone mit Dampf und einem dampfaktiven Dehydrierungskatalysator, der einen calcinierten Träger mindestens einer Aluminat-Spinell-Zusammensetzung, mindestens ein Metall der Gruppe VIII, mit oder ohne einen Gehalt an mindestens einem Metall der Gruppe IA und (d) mindestens ein Metall aus der Zinngruppe, das unter Zinn, Germanium und Blei ausgewählt ist, umfaßt, unter Dehydrierungsbedingungen, wodurch ein Produkstrom mit einem Gehalt an Isobutan, n-Butan, Butenen, Isobuten und Butadien gebildet wird;

(2) Trennen des Produktstroms, um ein abgetrenntes Produkt mit einem Gehalt an Butenen und Isobuten und eine zurückzuführende Ausgangsmaterialzusammensetzung mit einem Gehalt an Isobutan, n-Butan, Butadien und restlichen Butenen zu bilden; und

(3) Rückführen des zurückzuführenden Ausgangsmaterials zusammen mit frischem Ausgangsmaterial in die Reaktionszone in Form eines vereinigten Ausgangsmaterials, das bis zu 20 Mol-% n-Butan und bis zu etwa 5 Mol-% Butadien enthält, wodurch ein kombinierter Produktstrom mit einer Butadienmenge entsteht, die im wesentlichen der Menge an Butadien, die ohne die Anwesenheit von Butadien im Ausgangsmaterial gebildet wird, entspricht.

2. Verfahren nach Anspruch 1, gekennzeichnet durch folgende weitere Stufen:

(4) Auftrennen des vereinigten Produktstroms unter Bildung einer zur Rückführung vorgesehenen Einsatzmaterialzusammensetzung mit einem Gehalt an Isobutan, n-Butan und Butadien und restlichen Butenen und eines Produkts mit einem Gehalt an Butenen und Isobuten; und

(5) Rückführen der zur Rückführung vorgesehenen Einsatzmaterialzusammensetzung zusammen mit frischem Einsatzmaterial in die Reaktionszone unter Bildung eines Stroms mit einem Gehalt an Isobutan, n-Butan, Butenen, Isobuten und Butadien sowie restlichen Butenen.

3. Verfahren nach Anspruch 2, dadurch gekennzeichnet, daß man serienmäßig die Stufen (4) und (5) wiederholt, bis die gewünschte Produktmenge erhalten ist.

4. Verfahren nach Anspruch 1, dadurch gekennzeichnet, daß es sich beim calcinierten Träger um Zinkaluminat-Spinell handelt, wobei es sich beim Metall der Gruppe VIII um Platin und beim Metall der Zinngruppe um Zinn handelt.


**Revendications**

1. Procédé de déshydrogénation de la charge d'alimentation d'isobutane et de n-butane pour produire des butènes, de l'isobutène et du butadiène, ce procédé étant caractérisé par:

(1) la mise en contact de cette charge d'alimentation dans une zone de réaction avec de la vapeur et un catalyseur de déshydrogénation actif en présence de vapeur constitué d'un support calciné d'au moins une composition de spinelle d'aluminium, d'au moins un métal du Groupe VIII avec ou sans au moins un métal du Groupe IA et au moins un métal de groupe de l'étain choisi parmi l'étain, le germanium et le plomb, dans des conditions de déshydrogénation, donnant ainsi naissance à un courant de produit comprenant de l'isobutane, du n-butane, des butènes, de l'isobutène et du butadiène;

(2) la séparation de ce courant de produits pour donner un produit séparé comprenant des butènes et de l'isobutène et une composition de charge d'alimentation de recyclage comprenant de l'isobutane, du n-butane, du butadiène et des butènes résiduels; et

(3) le recyclage de cette charge d'alimentation de recyclage en même temps qu'une charge d'alimentation fraîche dans la zone de réaction en une charge d'alimentation combinée qui contient jusqu'à 20 moles % de n-butane et jusqu'à 5 moles % de butadiène, formant ainsi un courant de produits combiné contenant une quantité de butadiène pratiquement équivalente à la quantité de butadiène produite en l'absence du butadiène de la charge d'alimentation.

2. Procédé selon la revendication 1, caractérisé en ce qu'il comprend en outre:

(4) la séparation de ce courant de produits combiné pour produire une composition de charge d'alimentation de recyclage comprenant de l'isobutane, du n-butane, et du butadiène et des butènes résiduels et un produit contenant des butènes et de l'isobutène; et

(5) le recyclage de cette composition de charge d'alimentation de recyclage en même temps qu'une charge d'alimentation fraîche dans la zone réactionnelle pour produire un courant comprenant de l'isobutane, du n-butane, des butènes, de l'isobutène et du butadiène et des butènes résiduels.

3. Procédé selon la revendication 2, caractérisé en ce qu'on répète en série les stades (4) et (5) jusqu'à obtention de la quantité de produit désirée.

4. Procédé selon la revendication 1, caractérisé en ce que ce support calciné est un spinelle d'aluminate de zinc, en ce que ce métal du Groupe VIII est le platine et en ce que ce métal du Groupe de l'étain est l'étain.